# EUROPEAN PATENT SPECIFICATION

(11) **EP 2 641 470 B1**
(45) Date of publication and mention of the grant of the patent: **25.11.2020**
(21) Application number: 13166036.7
(22) Date of filing: 29.08.2007
(51) Int. Cl.: A01N 43/80, A01N 43/40, A01N 25/02, A01P 1/00

(54) **Microbicidal preparations based on 1,2-benzisothiazolin-3-one**
Mikrobizide Präparate auf der Basis von 1,2-Benzisothiazolin-3-on
Préparation microbicides à base de 1,2-benzisothiazolin-3-one

(30) Priority: 21.09.2006 DE 102006045065
(43) Date of publication of application: 25.09.2013
(62) Divisional of application: 07115162.5
(73) Proprietor: Vink Chemicals GmbH & Co. KG, 21255 Kakenstorf (DE)
(72) Inventor: Beilfuss, Wolfgang, 22339 Hamburg (DE); Hahn, Gisela, 25486 Alveslohe (DE); Weber, Klaus, 20146 Hamburg (DE); Gradtke, Ralf, 25436 Tornesch (DE); Siegert, Wolfgang, 25479 Ellerau (DE)
(74) Representative: LKGLOBAL Lorenz & Kopf PartG mbB Patentanwälte

(56) References cited:
- EP-A1- 1 013 751
- WO-A1-94/16564
- JP-A- 11 012 109
- Anonymous: "Ethylenediamine (CAS N°: 107-15-3)", Sids Initial Assessment for 13th SIAM (6-9 Noviembre, 2001) , 14 September 2001 (2001-09-14), pages 1-166, XP002571689, Bern, Switzerland Retrieved from the Internet: URL:http://www.chem.unep.ch/irptc/sids/oec dsids/Ethylenediamine.pdf [retrieved on 2010-02-12]

## Description

The present invention relates to microbicidal preparations based on 1,2-benzisothiazolin-3-one.

Preservative preparations which comprise isothiazolin-3-ones (isothiazolone) in combination with derivatives of 2-mercaptopyridine N-oxide (pyrithione below) are known, inter alia, for use as pack preservatives. For example, a combination of 1,2-benzisothiazolone with the zinc salt of 2-mercaptopyridine N-oxide (zinc pyrithione) in water-based emulsions or dispersions is known. However, zinc pyrithione is virtually insoluble in water and organic solvents and therefore cannot be formulated with 1,2-benzisothiazolone to give clear, homogeneous concentrates. Since they have a tendency to form inhomogeneous phases and are difficult to dose, dispersions are preferably not used as pack preservatives.

EP 1 013 751 A1 discloses, in the examples, a water-soluble combination prepared from two components for use as cutting fluid which comprises 1,2-benzisothiazolone, the sodium salt of 2-mercaptopyridine N-oxide (sodium pyrithione), ethylenediamine, and sodium carbonate and sodium bromide and which is present at a pH of 11.8. Due to the content of ethylenediamine, combinations of this type are burdened with disadvantages. For example, ethylenediamine is comparatively volatile and has an unpleasant odour. Via the gas phase, it leads to a strongly alkaline medium in the surrounding area (i.e. to the risk of corrosion, e.g. for aluminium-containing materials) and can lead to discolorations in preparations. Nitrosamine formation from ethylenediamine derivatives which may form from reactions with further ingredients of microbicidal preparations is not ruled out.

DE 100 40 814 A1 discloses a biocide composition with a content of a pyrithione as biocidal active ingredient which is characterized in that it comprises an iodoalkyl carbamate or a 2-alkylisothiazolin-3-one as a further biocidal active ingredient. Apart from the already described disadvantages of zinc pyrithione preferred according to DE 100 40 814 as pyrithione, the use of 2-n-octylisothiazolone preferred as 2-alkylisothiazolone is also associated with disadvantages. 2-n-Octylisothiazolone is virtually water-insoluble and is thus not homogeneously and clearly soluble and distributable in water-containing compositions, which, as mentioned, is required for pack preservatives. Added to this is the fact that it has been found that 2-alkylisothiazolones are of only limited stability in alkaline medium, they are degraded and are then insufficiently microbicidally effective.

DE 195 34 532 describes combinations of 2-n-octylisothiazolone with sodium pyrithione and solubility promoters. However, the amount of solubility promoter required when using alkylisothiazolone for preparing a clear solution is undesirably high.

Moreover, preparations are known which comprise formaldehyde or formaldehyde donor compounds. Preparations of this type have high microbicidal effectiveness. Preparations which definitely require the presence of formaldehyde and formaldehyde donor compounds for the microbicidal effect are undesired for certain applications for reasons of toxicity. This is also true for the algicidal triazines, which are prescribed according to DE 102 37 264 A1, which are N²-, N⁴-, N⁶-trialkyl-1,3,5-triazines.

It was an object of the invention to provide preservative preparations which
- are microbicidally effective over a wide pH range, including in particular in the alkaline pH range,
- have a high stability, including a high stability as concentrate, colour stability, low-temperature stability, active ingredient stability at various pH values and temperatures,
- are cost-effective in use,
- can be combined with alcohols which are effective in the vapour phase,
- are acceptable for customers, authorities and rating agencies (this requires a low allergy potential) and
- can be formulated as clear concentrates and clear use solutions.

According to the invention, it has now been found that these and other objects are achieved by a preservative preparation which comprises
a) 1 to 20% by weight of at least one of 1,2-benzisothiazolin-3-one and its halogen-free derivatives, wherein 1,2-benzisothiazolin-3-one and the halogen-free derivatives are given by the formula: 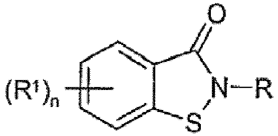 in which R may be H or C₁-C₁₀-alkyl, R¹ may be hydroxy, C₁-C₁₀-alkyl or C₁-C₁₀-alkoxy and n can be 0 to 4; if two or more radicals R¹ are present, then they can be identical or different,
b) 1 to 20% by weight of 2-mercaptopyridine N-oxide sodium salt, and
c) 0.3 to 10% by weight of at least one alkalinizing agent,
and comprises more than 30% by weight of water,
wherein the weight ratio of component a) to component b) is 1:2 to 2:1,
where the preparation
(i) has a pH of at least 12 and
(ii) is free from ethylenediamine.

It is clear to the person skilled in the art that components a) to c) prescribed according to the invention can react with one another. For example, it can lead to salt formation of the a) 1,2-benzisothiazolone and/or b) of the 2-mercaptopyridine N-oxide with c) alkalinizing agent.

The invention is based, inter alia, on the fact that it has been found that 1,2-benzisothiazolones can be formulated at high pH values and in the presence of 2-mercaptopyridine N-oxide to give clear preparations. This does not necessarily depend on the presence of solubility promoters, for example aromatic alcohols, especially in the case of comparatively low contents of a) and b). In a preferred embodiment, the preparation according to the invention is free from 2-alkylisothiazolones, as are described, for example, in combination with pyrithiones in DE 100 40 814 A1. 2-Alkylisothiazolones are not storage-stable over a long period, particularly at higher pH values, something which is shown in the examples of the present application.

In a further preferred embodiment, the preparation according to the invention is free from formaldehyde and formaldehyde donor compounds which, although highly effective in the gas phase, are toxic and irritate the eyes, the respiratory organs and the skin. Preparations according to the invention are characterized by the fact that they are exceptionally effective even in the absence of formaldehyde and formaldehyde donor compounds.

In a further preferred embodiment, preparations according to the invention are free from algicidal triazines as are known, for example, from DE 102 37 264 in combination with isothiazolones. The use of algicidal triazines is associated with disadvantages because algicidal triazines have virtually no bactericidal or fungicidal effectiveness, they are superfluous in pack preservatives, furthermore they are comparatively expensive and virtually water-insoluble.

In a further preferred embodiment, preparations according to the invention are free from quaternary ammonium compounds. Although quaternary ammonium compounds, as known from DE 101 44 187, are exceptionally effective even at high pH values and highly resistant to being washed out, the use of quaternary ammonium compounds is associated with disadvantages because they have a tendency towards foaming and are water-soluble only to a limited extent in strongly alkaline aqueous media. When using the preparation according to the invention, e.g. in household products, quaternary ammonium compounds are incompatible with the anionic surfactants customary therein.

In addition, preparations according to the invention are preferably free from iodopropynylbutyl compounds. Iodopropynylbutyl compounds are very slightly soluble in water, sensitive to hydrolysis and unstable in alkaline medium. They contribute to the (undesired) AOX content of a preparation and have a tendency towards discolorations.

Furthermore, preferred preparations according to the invention are free from the 1,3,5-triazine-2,4,6-tris-alkylaminocarboxylic acid derivatives according to DE 41 38 090 A1. The triazinecarboxylic acid derivatives of DE 41 38 090 are corrosion inhibitors with low bactericidal and fungicidal effectiveness compared to the benzisothiazolone or pyrithione compounds according to the invention. For example, the triazine derivative Becrosan 2126 (a triazine alkanolamide) is insufficiently effective against bacteria and fungi. Substances containing NH groups, such as the triazine carboxylic acid derivatives of DE 41 38 090, can react with nitrite to give nitrosamines. Corrosion inhibitors are not required in the presently claimed compositions (pack preservation).

Finally, preferred preparations according to the invention are free from hydrogencarbonate, bromide and/or carbonate. The preparations are free from ethylenediamine independently of the weight ratio a): b).

The preparation comprises more than 50% by weight of water, preferably 60 to 90% by weight of water, such as 80 to 85% by weight of water. Preparations with a water content of more than 77% by weight, such as more than 80% by weight, for example more than 82% by weight, are preferred here. The pH of the preparation is at least 12, preferably at least 12.2, more preferably 12.6 to 13.6, in particular 12.8 to 13.3.

### a) Benzisothiazolin-3-one

Component a) of the preparations according to the invention is chosen from 1,2-benzisothiazolin-3-one, its derivatives and mixtures thereof. 1,2-Benzisothiazolone and preferred derivatives are given by the formula: 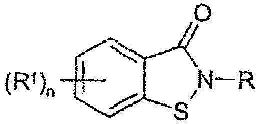 in which R may be H or C₁-C₁₀-alkyl, R¹ may be hydroxy, C₁-C₁₀-alkyl or C₁-C₁₀-alkoxy and n can be 0 to 4; if two or more radicals R¹ are present, then they can be identical or different. Compounds of this type are disclosed, inter alia, in WO 01/92444 A1. In a preferred embodiment, R is H. The derivatives are halogen-free. A particularly preferred component a) is 1,2-benzisothiazolin-3-one (i.e. n is zero and R is H).

The amount of component a) is 1 to 20% by weight, preferably 3 to 17% by weight, more preferably 5 to 15% by weight, such as 6 to 12% by weight, for example about 7.5 to 9.5% by weight.

When calculating the amounts of components a), b) and c) according to the present description of the invention, it should be taken into consideration that 1,2-benzisothiazolone and/or 2-mercaptopyridine N-oxide can form salt(s) with the alkalinizing agent in the preparation or can be formulated as salt(s) to give the preparation. Examples of salts of 1,2-benzisothiazolone are alkali metal, alkaline earth metal and amine salts or quaternary ammonium salts, such as Na, K, Li, Ca, Mg, ammonium, 2-hydroxyethylammonium and triethylammonium salts and mixtures thereof. Further examples of such salts are substoichiometric combinations of 1,2-benzisothiazolone and alkalinizing agents (which are reacted with 1,2-benzisothiazolone to give the corresponding salts). Particular preference is given to 1,2-benzisothiazolone, its sodium salt and its potassium salt.

When calculating the amount of 1,2-benzisothiazolin-3-one in the preparation, the amount of alkalinizing agent which has possibly led to salt formation is not taken into consideration, i.e. component a) when calculating the amount is treated as though no salt formation with the alkalinizing agent has taken place. This takes into account the fact that component a) of a preparation according to the invention is usually added as 1,2-benzisothiazolone and not as its salt.

### b) 2-Mercaptopyridine N-oxide

As component b) 2-mercaptopyridine N-oxide with the formula: 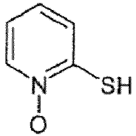 is used. Compound b) is used as the sodium salt, which can be used, for example, as a 40% strength aqueous solution.

In a particularly preferred embodiment, component b) is added as salt, for example as sodium pyrithione. Accordingly, if the 2-mercaptopyridine N-oxide in the preparation is in the form of the salt, when stating the amount of component b), this is taken into consideration in as far as only the amount of alkalinizing agent present over and above this salt formation of 2-mercaptopyridine N-oxide is taken into consideration when calculating the amount of the alkalinizing agent component c).

The amount of component b) in the preparation according to the invention is 1 to 20% by weight, preferably 2 to 15% by weight, more preferably 3 to 12% by weight, such as 4 to 10% by weight, for example 5 to 8% by weight.

The weight ratio of component a) to component b) is 1:2 to 2:1.

### c) Alkalinizing agent

Examples of alkalinizing agents are alkali metal hydroxides such as NaOH, KOH, LiOH, alkaline earth metal hydroxides, amines, for example alkanolamines, such as ethanolamine, alkali metal carbonates, alkali metal hydrogencarbonates and alkali metal silicates (such as waterglass).

Alkalinizing agents used in preference according to the invention are alkali metal hydroxides, where sodium hydroxide, potassium hydroxide or a mixture of sodium hydroxide and potassium hydroxide are particularly preferred as component c). In one embodiment in which a mixture consisting of sodium hydroxide and potassium hydroxide is used as alkalinizing agent, potassium hydroxide constitutes preferably 20 to 95% by weight of the mixture, more preferably 50 to 90% by weight, in particular 70 to 85% by weight, such as about 80% by weight (accordingly, the fraction of sodium hydroxide in the mixture is 5 to 80% by weight, 10 to 50% by weight, 15 to 30% by weight, such as about 20% by weight).

The amount of component c) in preparations according to the invention is 0.3 to 10% by weight, preferably 0.5 to 8% by weight, in particular 1 to 7% by weight, such as 1.5 to 6% by weight, for example 2.0 to 4.5% by weight.

When preparing a preparation according to the invention, it is not absolutely necessary that component c) is added separately, but it is also possible to ensure the high pH of the preparation prescribed according to the invention by introducing alkalinizing agent with component a) and/or component b) by, for example, using a salt of 1,2-benziso-thiazolin-3-one, (which is not preferred, but possible) or by using a salt of 2-mercaptopyridine N-oxide (which, in a preferred embodiment, is actually the case, in particular sodium pyrithione).

In one particular embodiment, 2-mercaptopyridine N-oxide is present as sodium salt, and equimolar amounts of component a) and component c) are present in the preparation. In a further embodiment which is preferred, a molar excess of alkalinizing agent is present in the preparation (besides sodium salt of 2-mercaptopyridine N-oxide), based on the amount of component a). An excess of alkalinizing agent is, in combination with a high pH, a particularly good prerequisite for the formulation of a stable liquid concentrate. Also particularly advantageous is the choice of the alkalinizing agents NaOH, KOH or a mixture thereof.

Preferred preparations according to the invention also comprise d) one or more aromatic alcohols, which makes a further contribution to the low-temperature stability and to the microbicidal effectiveness of the gas phase. Suitable aromatic alcohols are chosen from (i) aryloxyalkanols (glycol monoaryl ethers), (ii) arylalkanols and (iii) oligoalkanol aryl ethers.
(i) Aryloxyalkanols used according to the invention correspond to the formula Ar-O-(CHR)ₙ-OH where R = independently H (when n ≥ 2) or C₁ - to C₆-alkyl, where n is an integer and preferably 2 to 10, more preferably 2 to 6 and in particular 2 or 3. Whereas the group Ar can be a ring-substituted or unsubstituted aryl group, unsubstituted aryl, e.g. phenyl or naphthyl, are preferred. Examples of aryloxyalkanols used according to the invention are phenoxyethanol and phenoxypropanols. Preferred phenoxypropanols are 1-phenoxypropanol-2, 2-phenoxypropanol-1 or mixtures thereof, and 3-phenoxypropanol-1.
(ii) Arylalkanols used according to the invention have the formula Ar-(CHR)ₙ-OH where R = independently H or C₁- to C₆-alkyl, where n is an integer and preferably 1 to 10, more preferably 1 to 6 and in particular 1, 2, 3 or 4. While the group Ar may be a ring-substituted or unsubstituted aryl group, unsubstituted aryl, e.g. phenyl or naphthyl, are preferred. Examples of arylalkanols are 3-phenylpropanol-1, phenylethyl alcohol, veratryl alcohol (3,4-dimethoxyphenylmethyl alcohol), benzyl alcohol and 2-methyl-1-phenyl-2-propanol. (iii) Oligoalkanol aryl ethers include, for example, phenoxy-di-, -tri- and -oligoethanol and phenoxy-di-, -tri- and -oligopropanol. Besides phenoxyethanol, particularly preferred aromatic alcohols include phenoxypropanols, benzyl alcohol and mixtures thereof.

Preferred preparations according to the invention comprise 1 to 25% by weight, preferably 3 to 20% by weight, more preferably 5 to 15% by weight, such as 7 to 13% by weight, for example about 10% by weight, of component d) .

In a particularly preferred embodiment, the preparation is prepared from
a) 7 to 8% by weight of benzisothiazolone
b) 7 to 8% by weight of 2-mercaptopyridine N-oxide sodium salt,
c) about 2.0% by weight of sodium hydroxide and water as the remainder.

Alternative particularly preferred embodiments are parepared from
a) about 9% by weight of benzisothiazolone,
b) 5 to 6% by weight of 2-mercaptopyridine N-oxide sodium salt,
c) about 4.5% by weight of potassium hydroxide (or a mixture providing 3.6% by weight of potassium hydroxide and 0.9% by weight of sodium hydroxide) and
water as the remainder.

These preparations have very good storage stability over at least three months.

The invention is based, inter alia, on the fact that it has been found that 1,2-benzisothiazolones form water-soluble salts with alkali metal hydroxides, whereas 2-alkylisothiazolones, such as 2-n-octylisothiazolone, do not form salts with alkali metal hydroxides, are also virtually water-insoluble in an alkaline medium and, moreover, are unstable. Because of the high pH of preparations according to the invention, good storage stability is achieved which, even in the presence of solubility promoters, is not achieved at a lower pH.

The preparations according to the invention are characterized by the fact that they have good solubility and distributability in products with a high water content, and a high acceptance by customers and rating agencies. In addition, the components work synergistically together. Furthermore, preparations according to the invention have good storage stability. Moreover, the invention relates to a process for preparing the preparations according to the invention in which component b) (optionally as aqueous dispersion) is initially introduced into water, then component a) is added and then component c) and - if present - finally component d).

In an alternative embodiment of the process, component a) is initially introduced into water, then component c) is added and then component b), and then the mixture can be diluted to the desired concentration with water.

Moreover, the invention relates to the use of the preparation for preventing or reducing the microbicidal attack of a composition, in particular for the preservation and conservation of water-based products, such as cosmetic or pharmaceutical products, household products or technical products.

In addition, the invention relates to a method of preventing or reducing microbicidal attack of a composition in which the composition (for example a water-based product) is treated with an effective amount of the preparation according to the invention.

Furthermore, the invention relates to a preserved and/or conserved product, for example a water-based product, which comprises the preparation according to the invention.

Examples of further active ingredients which can be used in preparations according to the invention are the preservative active ingredients from Annexe 6 of the Cosmetics Ordinance, alcohols such as ethanol, propanol, polyols or derivatives thereof, for example butylene glycol, pentanediol-1,2, hexanediol-1,2, octanediol-1,2, decanediol-1,2, octoxyglycerol (2-ethylhexyl glycerol ether), octylglycerol, dodecyl-glycerol, glycerol monoesters such as glycerol monolaurate, glycerol caprylate, glycerol caprate, N-acylamino acids or derivatives thereof, such as N-octanoylglycine, alkali metal sulphite salts, alkali metal bisulphite salts or mixtures of these substances.

Preferred biocidal active ingredients are organohalogen compounds such as bronopol, iodopropynyl butylcarbamate, dibromodicyanobutane, dichlorobenzyl alcohol, chlorofenesin, carboxylic acids or salts thereof such as formic acid, sorbic acid, salicylic acid, benzoic acid, dehydracetic acid, undecylenic acid, phenols such as parabens or salts thereof (e.g. methyl-, ethyl-, propyl- and butylparaben), o-phenylphenol, p-chloro-m-cresol, aldehydes such as formaldehyde, glutardialdehyde, succinaldehyde, aldehyde donor compounds such as formaldehyde donor compounds (e.g. O- or N-formals such as ethylene glycol bishemiformal, benzyl alcohol bishemiformal, Grotan BK, Grotan OX, Grotan OF, Grotan OK, tetramethylolacetylenediurea, dimethyloldimethylhydantoin, diazolidinylurea, dimethylolurea), succinaldehyde donor compounds (e.g. dimethoxytetrahydrofuran), glutardialdehyde donor compounds (e.g. alkoxydihydropyrans, alkoxytetrahydropyrans), isothiazolones such as methyl-, chloromethyl-, octylisothiazolone, cationic compounds and quaternary ammonium compounds such as benzalkonium chloride, benzethonium chloride, Vantocil IB, Bardac 22, chlorhexidine salts, alexidine salts, peroxides such as H₂O₂, methyl ethyl ketone peroxide, t-butyl hydroperoxide, peracetic acid or mixtures thereof.

Examples of functional additives are complexing agents such as EDTA, NTA, pH correctors or buffers such as citrates, phosphates, antioxidants such as vitamin E, phenol derivatives, low-temperature stabilizers such as glycols, glycol ethers, solubility promoters such as alcohols, glycols, glycol ethers, e.g. ethylene glycol, triethylene glycol, 1,2-propylene glycol, crystallization preventors, viscosity modifiers, thickeners or salts thereof or mixtures of these substances.

The advantages of the invention are particularly evident from the following examples.

### Example 1

### Decomposition of 2-n-octylisothiazolone at a pH of about 13

1.1% by weight of Kathon 893F (2-n-octylisothiazolone, 45% strength in glycol), 48.89% by weight of methanol and 50% by weight of sodium hydroxide solution (c = 0.5 mol/1) were used to prepare a mixture with a pH of about 13 whose active ingredient content (in % by wt.) was monitored using HPLC over a period of 35 days. Storage took place at 20°C. The results are shown below.

| Days | 2-n-Octylisothiazolone |
|---|---|
| 0 | 0.53 |
| 2 | 0.43 |
| 4 | 0.36 |
| 7 | 0.29 |
| 11 | 0.23 |
| 21 | 0.13 |
| 35 | 0.07 |

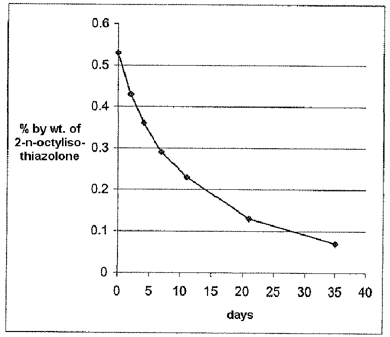

This shows that 2-alkylisothiazolones, such as 2-n-octylisothiazolone, are not stable under alkaline conditions.

### Example 2

The following preparations were prepared by mixing the stated components. The quantitative data in the table refer to the amounts of active constituent. 2-Mercaptopyridine N-oxide was used as the sodium salt sodium pyrithione (40% strength aqueous solution), 1,2-benzisothiazolinone was used as 85% strength hydrous solid. Potassium hydroxide and sodium hydroxide were in each case used as 45% strength aqueous solution.

| | I | II (comparison) | III | IV (comparison) |
|---|---|---|---|---|
| 1,2-Benzisothiazolin-3-one | 9.0 | 9.0 | 7.6 | 9.0 |
| Sodium pyrithione | 5.6 | 5.6 | 7.6 | 5.6 |
| Potassium hydroxide | 4.5 | 3.6 | - | - |
| Sodium hydroxide | - | 0.9 | 2.0 | - |
| Phenoxyethanol | - | - | - | 10.0 |
| Water | ad 100 | ad 100 | ad 100 | ad 100 |
| Solubility and appearance after preparing the preparation | Dissolved after 10 min, clear, brownish-yellow solution | Dissolved after 10 min, clear brownish-yellow solution | After filtration, clear, orange-yellow solution | Not dissolved, cloudy, yellowish solution with a lot of sediment |
| Gardner colour number after preparation | 4.4 | 4 | 3.8 | |
| pH (pH paper) after preparation | 13-14 | 13-14 | 12.2 | 8-9 |
| Appearance after storage for 2 days at -5°C | frozen | frozen | | Without change |
| Appearance after storage for 2 days at +4°C | Without change | frozen | | |
| Appearance after storage for 2 days at 25°C | Without change | Without change | Very good storage stability over at least three months at +40°C | |

## Claims

1. Preservative preparation which comprises
a) 1 to 20% by weight of at least one of 1,2-benzisothiazolin-3-one and its halogen-free derivatives, wherein the 1,2-benzisothiazolin-3-one and the halogen-free derivatives are given by the formula: 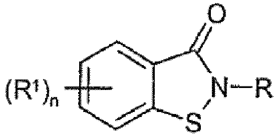 in which R may be H or C₁-C₁₀-alkyl, R¹ may be hydroxy, C₁-C₁₀-alkyl or C₁-C₁₀-alkoxy and n can be 0 to 4; if two or more radicals R¹ are present, then they can be identical or different,
b) 1 to 20% by weight of 2-mercaptopyridine N-oxide sodium salt, and
c) 0.3 to 10% by weight of at least one alkalinizing agent,
and comprises more than 30% by weight of water,
wherein the weight ratio of component a) to component b) is 1:2 to 2:1,
where the preparation
(i) has a pH of at least 12 and
(ii) is free from ethylenediamine.

2. Preparation according to Claim 1 which comprises more than 50% by weight of water.

3. Preparation according to Claim 1 or 2 which is free from N-alkylisothiazolin-3-one.

4. Preparation according to one of the preceding claims, **characterized in that** its pH is at least 12.2.

5. Preparation according to one of the preceding claims, **characterized in that** component a) is 1,2-benzisothiazolin-3-one.

6. Preparation according to one of the preceding claims, **characterized in that** the amount of component a) is 3 to 17% by weight.

7. Preparation according to one of the preceding claims, **characterized in that** the amount of component b) is 2 to 15% by weight.

8. Preparation according to one of the preceding claims, **characterized in that** the alkalinizing agent is an alkali metal hydroxide, where potassium hydroxide, sodium hydroxide or a mixture of sodium hydroxide and potassium hydroxide is particularly preferred as component c).

9. Preparation according to one of the preceding claims, **characterized in that** the amount of component c) is 0.5 to 8% by weight.

10. Preparation according to one of the preceding claims, **characterized in that** it also comprises
d) 1 to 25% by weight of one or more of phenoxyethanol, phenoxypropanols, benzyl alcohol and mixtures thereof.

11. Preparation according to Claim 10, **characterized in that** the amount of component d) is 3 to 20% by weight.

12. Process for preparing the water-containing preparation according to one of Claims 2 to 11, in which component a) is initially introduced into water, then component c) is added and then component b), and then the mixture can optionally be diluted to the desired concentration with water.

13. Use of the preparation according to one of Claims 1 to 11 for preventing or reducing the microbicidal attack of a composition.

14. Method of preventing or reducing the microbicidal attack of a composition in which the composition is treated with an effective amount of the preparation according to one of Claims 1 to 11.

## Patentansprüche

1. Konservierungsmittelzubereitung, die umfasst
a) 1 bis 20 Gew.-% mindestens eines von 1,2-Benzisothiazolin-3-on und seinen halogenfreien Derivaten, wobei das 1,2-Benzisothiazolin-3-on und die halogenfreien Derivate gegeben sind durch die Formel 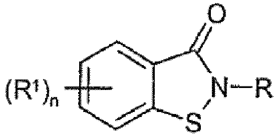 in der R für H oder C₁-C₁₀-Alkyl stehen kann, R¹ für Hydroxy, C₁-C₁₀-Alkyl oder C₁-C₁₀-Alkoxy stehen kann und n für 0 bis 4 stehen kann; wenn zwei oder mehr Reste R¹ vorhanden sind, können sie gleich oder verschieden sein,
b) 1 bis 20 Gew.-% Natriumsalz von 2-Mercaptopyridin-N-oxid, und
c) 0,3 bis 10 Gew.-% mindestens eines Alkalisierungsmittels,
und mehr als 30 Gewichtsprozent Wasser umfasst,
worin das Gewichtsverhältnis der Komponente a) zur Komponente b) 1:2 bis 2:1 beträgt,
wobei die Zubereitung
(i) einen pH-Wert von mindestens 12 aufweist und
(ii) frei von Ethylendiamin ist.

2. Zubereitung gemäß Anspruch 1, die mehr als 50 Gew.-% Wasser umfasst.

3. Zubereitung gemäß Anspruch 1 oder 2, die frei von N-Alkylisothiazolin-3-on ist.

4. Zubereitung gemäß einem der vorstehenden Ansprüche, **dadurch gekennzeichnet, dass** ihr pH-Wert mindestens 12,2 beträgt.

5. Zubereitung gemäß einem der vorstehenden Ansprüche, **dadurch gekennzeichnet, dass** die Komponente a) 1,2-Benzisothiazolin-3-on ist.

6. Zubereitung gemäß einem der vorstehenden Ansprüche, **dadurch gekennzeichnet, dass** die Menge der Komponente a) 3 bis 17 Gew.-% beträgt.

7. Zubereitung gemäß einem der vorstehenden Ansprüche, **dadurch gekennzeichnet, dass** die Menge der Komponente b) 2 bis 15 Gew.-% beträgt.

8. Zubereitung gemäß einem der vorstehenden Ansprüche, **dadurch gekennzeichnet, dass** das Alkalisierungsmittel ein Alkalimetallhydroxid ist, wobei als Komponente c) Kaliumhydroxid, Natriumhydroxid oder ein Gemisch aus Natriumhydroxid und Kaliumhydroxid besonders bevorzugt ist.

9. Zubereitung gemäß einem der vorstehenden Ansprüche, **dadurch gekennzeichnet, dass** die Menge der Komponente c) 0,5 bis 8 Gew.-% beträgt.

10. Zubereitung gemäß einem der vorstehenden Ansprüche, **dadurch gekennzeichnet, dass** sie auch
d) 1 bis 25 Gew.-% eines oder mehrerer von Phenoxyethanol, Phenoxypropanolen, Benzylalkohol und Mischungen davon
umfasst.

11. Zubereitung gemäß Anspruch 10, **dadurch gekennzeichnet, dass** die Menge der Komponente d) 3 bis 20 Gew.-% beträgt.

12. Verfahren zur Herstellung der wasserhaltigen Zubereitung gemäß einem der Ansprüche 2 bis 11, bei dem zunächst die Komponente a) in Wasser eingebracht wird, dann die Komponente c) hinzugegeben wird und dann die Komponente b), und dann die Mischung gegebenenfalls mit Wasser auf die gewünschte Konzentration verdünnt werden kann.

13. Verwendung der Zubereitung gemäß einem der Ansprüche 1 bis 11 zur Verhinderung oder Verringerung des mikrobiziden Angriffs auf eine Zusammensetzung.

14. Verfahren zur Verhinderung oder Verringerung des mikrobiziden Angriffs auf eine Zusammensetzung, bei dem die Zusammensetzung mit einer wirksamen Menge der Zubereitung gemäß einem der Ansprüche 1 bis 11 behandelt wird.

## Revendications

1. Préparation conservatrice qui comprend
a) de 1 à 20 % en poids d'au moins un parmi la 1,2-benzisothiazolin-3-one et ses dérivés non halogénés, la 1,2-benzisothiazolin-3-one et ses dérivés non halogénés étant représentés par la formule : 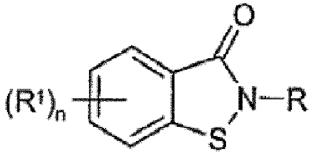 dans laquelle R peut représenter H ou un groupe alkyle en C₁ à C₁₀, R¹ peut représenter un groupe hydroxy, un groupe alkyle en C₁ à C₁₀ ou un groupe alcoxy en C₁ à C₁₀ et n peut représenter 0 à 4 ; si deux radicaux R¹ ou plus sont présents, alors ils peuvent être identiques ou différents,
b) de 1 à 20 % en poids de sel sodique de N-oxyde de 2-mercaptopyridine, et
c) de 0,3 à 10 % en poids d'au moins un agent alcalinisant,
et comprend plus de 30 % en poids d'eau,
dans laquelle le rapport pondéral du composant a) sur le composant b) est de 1:2 à 2:1,
où la préparation
(i) a un pH d'au moins 12 et
(ii) est exemptes d'éthylènediamine.

2. Préparation selon la revendication 1 qui comprend plus de 50 % en poids d'eau.

3. Préparation selon la revendication 1 ou 2 qui est exempte de N-alkylisothiazolin-3-one.

4. Préparation selon l'une des revendications précédentes, **caractérisée en ce que** son pH est d'au moins 12.2.

5. Préparation selon l'une des revendications précédentes, **caractérisée en ce que** le composant a) est la 1,2-benzisothiazolin-3-one.

6. Préparation selon l'une des revendications précédentes, **caractérisée en ce que** la quantité de composant a) est de 3 à 17 % en poids.

7. Préparation selon l'une des revendications précédentes, **caractérisée en ce que** la quantité de composant b) est de 2 à 15 % en poids.

8. Préparation selon l'une des revendications précédentes, **caractérisée en ce que** l'agent alcalinisant est un hydroxyde de métal alcalin, où l'hydroxyde de potassium, l'hydroxyde de sodium ou un mélange d'hydroxyde de sodium et d'hydroxyde de potassium est particulièrement préféré en tant que composant c).

9. Préparation selon l'une des revendications précédentes, **caractérisée en ce que** la quantité de composant c) est de 0,5 à 8 % en poids.

10. Préparation selon l'une des revendications précédentes, **caractérisée en ce qu'**elle comprend également
d) de 1 à 25 % en poids d'un ou plusieurs parmi le phénoxyéthanol, les phénoxypropanols, l'alcool benzylique et des mélanges de ceux-ci.

11. Préparation selon la revendication 10, **caractérisée en ce que** la quantité de composant d) est de 3 à 20 % en poids.

12. Procédé de préparation de la préparation contenant de l'eau selon l'une des revendications 2 à 11, dans lequel le composant a) est initialement introduit dans l'eau, puis le composant c) est ajouté, puis le composant b), et ensuite le mélange peut être éventuellement dilué à la concentration souhaitée avec de l'eau.

13. Utilisation de la préparation selon l'une des revendications 1 à 11 pour la prévention ou la réduction de l'attaque microbienne d'une composition.

14. Méthode de prévention ou de réduction de l'attaque microbienne d'une composition dans laquelle la composition est traitée avec une quantité efficace de la préparation selon l'une des revendications 1 à 11.
